# EUROPÄISCHE PATENTANMELDUNG

(11) **EP 0 943 337 A2**
(43) Veröffentlichungstag der Anmeldung: **22.09.1999**
(21) Anmeldenummer: 99101335.0
(22) Anmeldetag: 25.01.1999
(51) Int. Cl.: A61K 38/48

(54) **Verwendung von mindestens einem hydrolytischen Enzym und mindestens einem Flavonoid zur Behandlung von durch Hepatitis C Viren hervorgerufenen Erkrankungen**

(30) Priorität: 06.02.1998 DE 19804742
(71) Anmelder: MUCOS Pharma GmbH & Co., D-82538 Geretsried (DE)
(72) Erfinder: Stauder, Gerhard, Dr., 82538 Geretsried (DE); Ransberger, Karl, 82402 Seeshaupt (DE)
(74) Vertreter: Grünecker, Kinkeldey, Stockmair & Schwanhäusser Anwaltssozietät

(57) **Zusammenfassung**

Die Verwendung von mindestens einem hydrolytischen Enzym und mindestens einem Flavonoid zeigt bei der Behandlung von durch Hepatitis C Viren hervorgerufenen Erkrankungen bessere Wirkung als α-Interferon oder Ribavirin.

## Beschreibung

Die vorliegende Erfindung betrifft die Verwendung von mindestens einem hydrolytischen Enzym und mindestens einem Flavonoid zur Behandlung von durch Hepatitis C Viren hervorgerufenen Erkrankungen.

Hepatitis ist eine Entzündung der Leber, die aufgrund verschiedener Ursachen entstehen kann. Die akuten Hepatididen werden meist durch Viren hervorgerufen. Derzeit kennt man sechs verschiedene Typen von Hepatitisviren (A-E und G). Die Hepatitis C ist eine Viruserkrankung bei der es zu einer Persistenz des Hepatitis C Virus oder einer chronischen Hepatitis kommen kann. Diese chronische Hepatitis C entwickelt sich bei ungefähr 50% der infizierten Individuen. Ungefähr 20% der chronisch an Hepatitis C erkrankten Individuen zeigen histologische Befunde der Leberzirrhose. Das Hepatitis C Virus spielt darüber hinaus wahrscheinlich eine wichtige Rolle bei der Entwicklung hepatozellulärer Karzinome. Diese Komplikationen führen häufig zum Tode. Die medikamentöse Behandlung der Hepatitis C ist immer noch im Versuchsstadium. Üblicherweise werden an Hepatitis C erkrankte Individuen durch diätische Maßnahmen behandelt um eine Schonung des Leberstoffwechsels hervorzurufen. Weiterhin wird der Einsatz von α-Interferon und Ribavirin in Erwägung gezogen. Diese Mittel zeigen allerdings häufig unerwünschte Nebeneffekte, und beide sind darüber hinaus auch sehr teuer. Beide Präparate zeigen weiterhin auf Dauer nur bei ca. 20% der Patienten Wirkung.

Der vorliegenden Erfindung lag daher das technische Problem zugrunde, weitere Stoffe oder Kombinationen anzugeben, die eine verbesserte Wirkung bei der Behandlung von durch Hepatitis C Virus hervorgerufenen Erkrankungen zeigen.

Diese Aufgabe wird durch die Verwendung von mindestens einem hydrolytischen Enzym und mindestens einem Flavonoid gelöst.

Hydrolytische Enzyme werden unter dem Oberbegriff "Hydrolasen" zusammengefaßt. Diese Enzyme sind in der Lage Substrate durch Einbau von Wasser zu spalten. Sie bilden im Nomenklatursystem der IUBMB die dritte Enzymklasse. Zu ihnen gehören Esterasen, Glykosidasen, Etherhydrolasen, die große Gruppe der Peptidasen (Proteasen) sowie Enzyme, die Bindungen zwischen Kohlenstoff und Stickstoff (Amidasen, Amidinasen, Nitrilasen), Säureanhydridbindungen, Kohlenstoff-Kohlenstoffbindungen, Kohlenstoff-Halogenbindungen, Phosphor-Stickstoffbindungen, Schwefel-Stickstoffbindungen und Kohlenstoff-Phosphorbindungen unter Hydrolyse lösen.

Bevorzugt unter diesen Hydrolasen sind Proteasen. Diese Enzyme katalysieren die hydrolytische Spaltung der Peptidbindung in Proteinen und Peptiden. Proteasen umfassen Proteinasen und Peptidasen. Beispiele von Proteasen die in der vorliegenden Erfindung verwendet werden können sind Chymotrypsin, Elastase, Katepsine, Pepsin, Plasmin, Trypsin, Bromelain und Papain. Bevorzugt sind davon Bromelain, Trypsin und Papain.

Die erfindungsgemäß verwendeten bevorzugten Enzyme lassen sich kostengünstig aus den folgenden Rohmaterialien isolieren.

Bromelain ist ein proteolytisch wirksames Enzym aus dem Pressaft der Ananas und kann auch noch aus reifen Früchten isoliert werden.

Papain ist ein proteolytisches Enzym, das aus dem Milchsaft der unreifen, fleischigen Früchte des Melonenbaums Carica Papaya gewonnen wird. Reines Papain ist ein kristallines Polypeptid mit einem Molgewicht von 23.350, das aus einer Kette von 212 Aminosäureresten mit vier Disulfit-Brücken besteht; Sequenz und Raumstruktur sind bekannt. Papain wird vielfältig eingesetzt: aufgrund seiner protein-spaltenden Eigenschaff als "Fleischzartmacher" oder "Mürbesalz", zum Klären von Bier, zur Brot- und Hartkeksherstellung, in der Lederzubereitung, in der Textilindustrie, zum Entbasten von Seide und zur Verhinderung von Wollverfilzung, in der Tabakindustrie zur Qualitätverbesserung, zur Rückgewinnung von Silber aus verbrauchtem photographischem Material, ferner in der Bakteriologie zur Pepton-Gewinnung. In der Medizin dient Papain bereits zur Unterstützung der enzymatischen Verdauung, zur enzymatischen Wundreinigung und als Zusatz zu Zahnprothesereinigungsmitteln. Für spezielle Zwecke werden Papain-Präparate auch an Kunststoffpolymere oder Agarose Trägergebunden angeboten. Papain ist auch als Katalysator zur Synthese von Oligopeptiden verwendet worden.

Trypsin ist ein proteolytisches Enzym, das im Pankreas gebildet wird, und in Verbindung mit anderen Enzymen bereits therapeutisch eingesetzt wird. Es gehört zu den Serinproteinasen. Kristallines Trypsin hat einen Molgewicht von ca. 23.300, ist in Wasser, nicht aber in Alkohol löslich, besitzt ein Wirkungsoptimum bei pH 7 bis 9 und spaltet Peptidketten spezifisch Carboxyseitig der basischen Aminosäurereste L-Lysin und L-Arginin. Die räumliche Struktur des aus 223 Aminosäure bestehenden Trypsins ist bekannt.

Eine besonders gute Wirksamkeit zeigt sich bei der Verwendung eine Kombination der Enzyme Bromelain, Papain und/oder Trypsin. Diese Enzyme werden erfindungsgemäß in Kombination mit mindestens einem Flavoid verwendet.

Flavonoide, die in allen höheren Pflanzen vorkommen, sind wichtigte Phenylpropanderivate mit dem C15 Grundgerüst des Flavans. Sie liegen vorwiegend wasserlöslich in den Vakuolen pflanzlicher Zellen in glycosylisierter Form vor und sind häufig mit aliphatischen und/oder aromatischen Säuren verestert, z.B. mit Malonsäure bzw. Kaffeesäure. Nicht glycosylisierte lipophile Flavonoide treten als nicht flüchtige Komponenten in etherischen Ölen auf, akkumulieren im Holzparenchym oder werden auf die Epidermis von Blättern ausgeschieden. Die Aglyka der Flavonoide werden nach dem Oxidationsgrad ihres zentralen Pyranrings in folgende Klassen unterteilt: Anthocyanidine, Aurone, Catechine, Chalkone, Desoxyanthacyanidine, Flavonole, Flavanone, Flavolone, Flavone, Isoflavone und Leukoanthocyanidine. Ein bevorzugtes Flavonoid ist Rutosid, ein Pentahydroxyfiavonderivat mit einem Molgewicht von 610. Bevorzugt ist insbesondere Rutosid in der Form der sauren Natriumsalze. Es können aber auch synthetische Rutosidderivate wie Troxserutin und Monoxerutin eingesetzt werden. Außer von Rutosid können auch aus Ginko biloba gewonnene Präparate eingesetzt werden.

Es wird davon ausgegangen - ohne an eine Theorie gebunden zu sein -, daß ein Metabolit des Rutosid, wie Quercetin, die pharmakologische Wirkung entfaltet. Präparate aus Ginko biloba ergeben ähnliche Metabolite.

Neben der bemerkenswerten und unerwarteten Wirkung der Kombination aus mindestens einem hydrolytischen Enzym und mindestens einem Flavonoid bei der Behandlung von Hepatitis C Virus-Erkrankungen hat die kombinierte Verwendung weiterhin den Vorteil, daß auch bei einer längeren Anwendung keine schädigenden Nebenwirkungen auftreten.

Eine besonders gute Wirksamkeit hat die kombinierte Verwendung von 90 mg Bromelain, 48 mg Trypsin und 100 mg Rutosid.

Die erfindungsgemäße Kombination kann weiterhin zusammen mit anderen Virostatika verwendet werden. Insbesondere kann dabei α-Interferon und/oder Ribavirin verwendet werden.

α-Interferon ist ein Protein aus der Gruppe der Cytokine, das antivirale, aber virusunspezifische Aktivität aufweist. Bevorzugt werden 1 Mio. bis 6 Mio. I.E. α-Interferon 1 bis 7 mal pro Woche verwendet.

Ribavirin ist ein synthetisches Nukleosid mit breitem Wirkungsspektrum als Virostatikum. Bevorzugt werden 1000 mg bis 1500 mg Ribavirin täglich verwendet, insbesondere bevorzugt 1200 mg.

Die erfindungsgemäße Kombination kann weiterhin mit allen üblichen Hilfs- und/oder Trägerstoffen verwendet werden.

Als Hilfs- und Trägerstoffe kommen alle üblicherweise verwendeten Substanzen in Frage, z.B. Füllmittel, Bindemittel, Verdickungsmittel, Adsorptionsmittel, Trocknungsmittel, Gelbildner, Filmbildner, Sorptionsmittel, Gleitmittel, Formentrennmittel, Zerfallsbeschleuniger, Sprengmittel, Antioxidantien, Konservierungsmittel, Geschmacks- und Geruchskorrigentien sowie Färbemittel. Diese Substanzen können in den üblichen Mengen verwendet werden. Illustrative Beispiele sind Zucker und Zuckeralkohole wie Lactose, Saccharose, Traubenzucker, Mannitol, Sorbitol; makromolekulare Hilfsstoffe wie Stärken, Cyclodextrine, Gelatine, Tragant, Pektin, Cellulose, Methylcellulose, Polyacrylate, Polyvinylalkohol, Makrogele, Polyethylenoxide; oberflächenaktive Hilfsstoffe wie Natriumdodecylsulfat, Lecithin, Fettalkohole und Sterole; anorganische Hilfsstoffe wie Talkum, weißer Ton, Bentonit, TiO₂, CaHPO₄, CaCO₃, NaHCO₃, SiO₂; sowie organische Hilfsstoffe wie Vanillin, Dibutylphthalat, polyindon, gelbes Wachs, Shellack und Camaubawachs. Bevorzugt erfolgt die erfindungsgemäße Verwendung durch orale Applikation, jedoch sind auch alle anderen üblichen Applikationsformen möglich. Die folgenden Beispiele erläutern die Erfindung weiter.

80 an Hepatitis C erkrankte Patienten wurden in vier parallele Gruppen eingeteilt. Gruppe 1 erhielt oral dreimal täglich zwei Tabletten Phlogenzym (eingetragenes Warenzeichen). Jede Tablette Phlogenzym enthält 90 mg Bromelin, 48 mg Trypsin und 100 mg Rutosit.

Gruppe 2 erhielt α-Interferon, Gruppe 3 Ribavirin. Gruppe 4 erhielt eine leberunterstützende diätische Behandlung (Vitamine, Mineralstoffe). Jeder Patient wurde für 12 Wochen behandelt.

Keiner der Patienten war an Hepatitis A, B oder D oder einer anderen Leberkrankheit erkrankt.

Die Überwachung der Patientengruppen und damit der Wirksamkeit der Medikamentation erfolgte über die Verfolgung der Funktion der Lebertransaminasen AST (Aspartat-Aminotransferase), ALT (Alanin-Aminotransferase) und S-γ-GT (Serum-γ-Glutamyl-Transpeptidase).

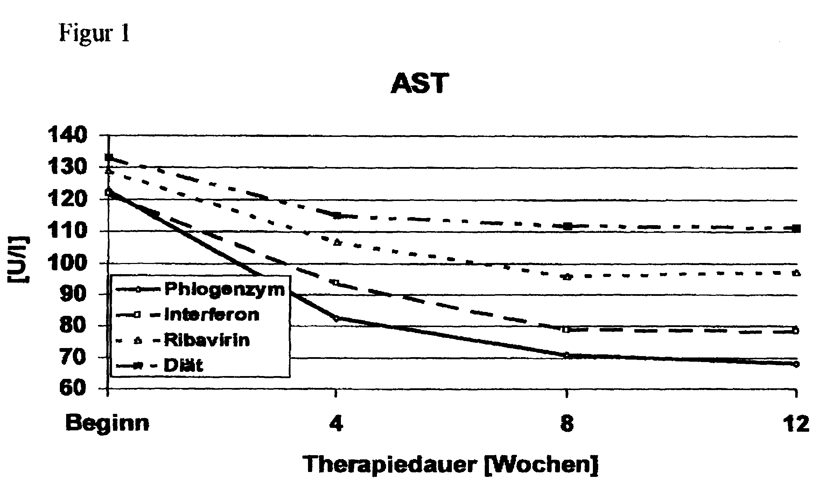

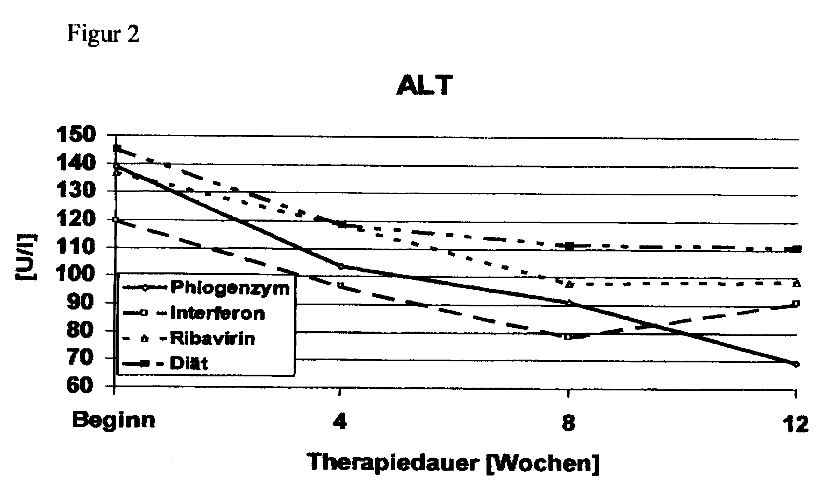

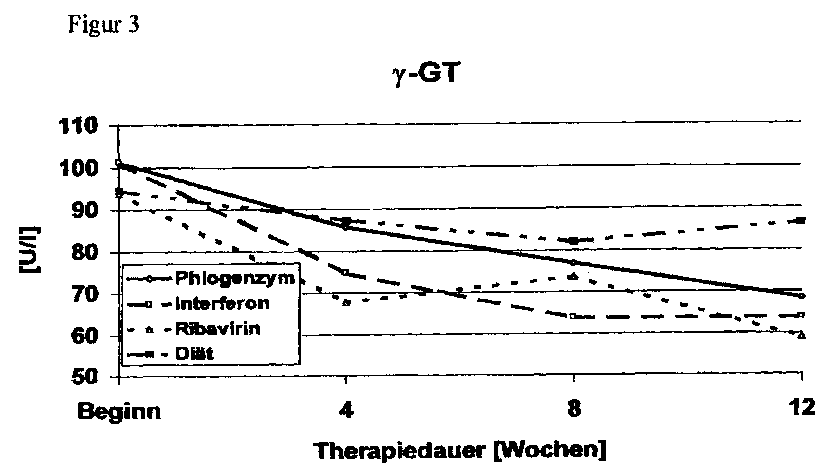

Diese Angaben sind in graphischer Form in den Figuren 1 bis 3 dargestellt.

Die Werte für die Funktion der Lebertransaminasen sanken in allen 4 Gruppen von pathologischen Bereichen. Die Verbesserung in der Gruppe, die die diätische Behandlung erhielt war dabei am geringsten. Auch die Gruppe, die nur Ribavirin erhielt zeigte nur eine unwesentliche Verbesserung. Eine wesentliche Verbesserung wurde nur in den Gruppen erzielt, die α-Interferon bzw. Phlogenzym erhielten. Allerdings zeigt nur die Gruppe der Phlogenzym-Patienten eine kontinuierliche Verbesserung. Bei der Verwendung von α-Interferon zeigte sich jeweils nach 8 Wochen wieder eine Verschlechterung bzw. mindestens Stagnation. Eine gute Wirksamkeit zeigte auch die Kombination von Phlogenzym und α-Interferon bzw. Ribavirin.

Eine Extrapolation dieser Ergebnisse deutet darauf hin, daß eine längere Behandlung mit Phlogenzym eine noch deutlichere Verbesserung herbeiführen könnte.

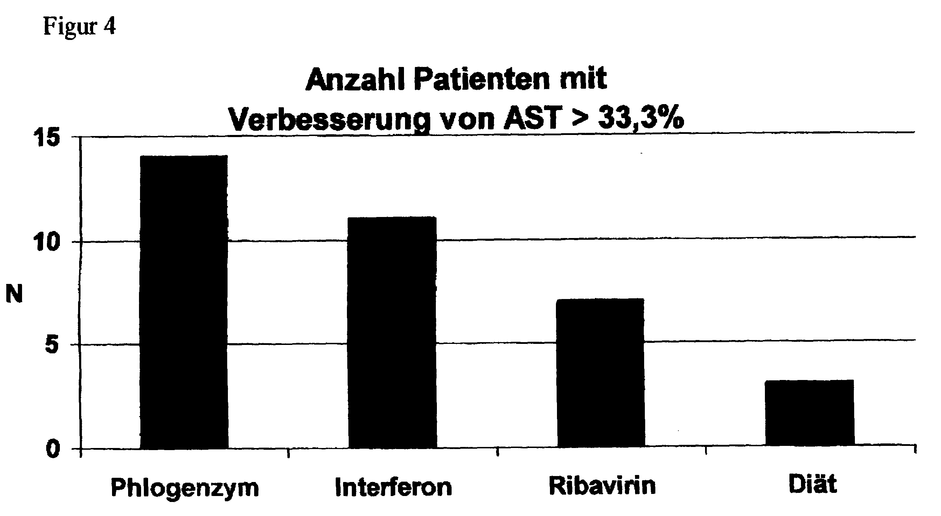

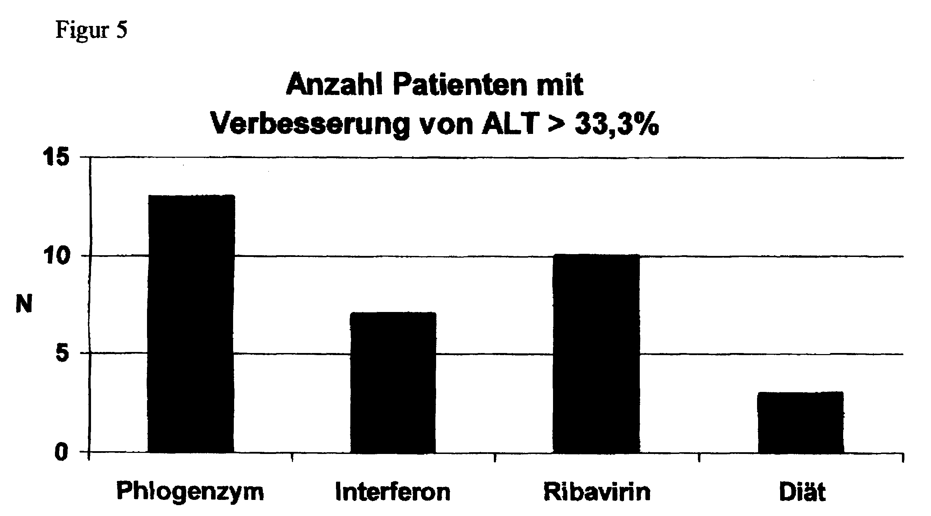

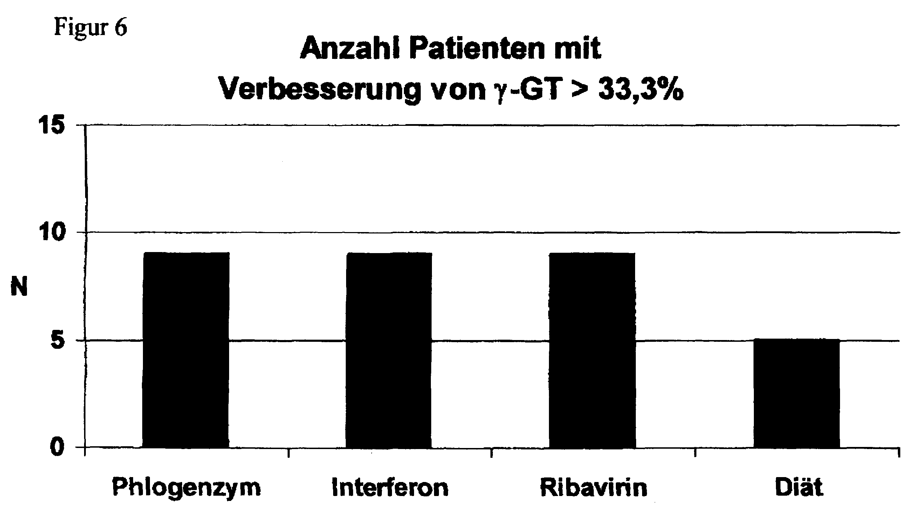

Berechnet man die Anzahl der Patienten aus jeder Gruppe, deren Werte sich um mehr als 1/3 vom Ausgangswert verbessert haben, so wird der Unterschied zwischen den 4 Gruppen noch deutlicher. Diese Berechnungen sind graphisch in den Figuren 4-6 dargestellt. Es wird deutlich, daß die Behandlung mit Phlogenzym die effektivste Behandlung darstellt.

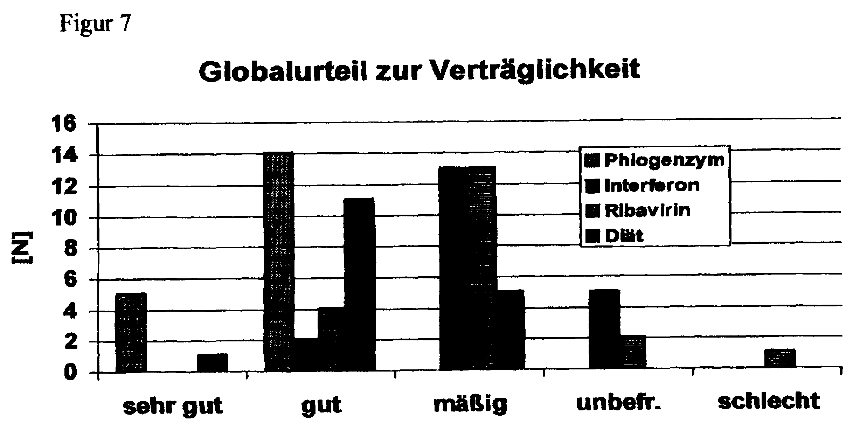

Wird zusätzlich noch die Bewertung der Verträglichkeit (dargestellt in Figur 7) zur Beurteilung herangezogen, wird der Unterschied zwischen der Behandlung mit Phlogenzym und den anderen Monotherapien noch deutlicher. Am Ende der Therapie bewerten alle Patienten die mit Phlogenzym behandelt worden waren die Verträglichkeit mit "sehr gut" oder "gut". PCR Messungen haben darüberhinaus gezeigt, daß bei den Phlogenzym-Patienten eine Verringerung der Virusbelastung um 50% zu beobachten war.

## Patentansprüche

1. Verwendung von mindestens einem hydrolytischen Enzym und mindestens einem Flavonoid zur Behandlung von durch Hepatitis C Viren hervorgerufenen Erkrankungen.

2. Verwendung gemäß Anspruch 1, **dadurch gekennzeichnet,** daß als hydrolytisches Enzym ein proteolytisches Enzym verwendet wird.

3. Verwendung nach Anspruch 2, **dadurch gekennzeichnet,** daß als proteolytisches Enzym Trypsin, Bromelain oder Papain oder eine Kombination von einem oder mehreren dieser Enzyme verwendet wird.

4. Verwendung nach mindestens einem der vorstehenden Ansprüche, **dadurch gekennzeichnet,** daß das Flavenoid Rutosid ist.

5. Verwendung nach mindestens einem der vorstehenden Ansprüche, **dadurch gekennzeichnet,** daß 20 bis 100 mg Bromelain, 10 bis 70 mg Trypsin und 80 bis 120 mg Rutosid pro Dosiereinheit verwendet werden.

6. Verwendung nach mindestens einem der vorstehenden Ansprüche, **dadurch gekennzeichnet,** daß zusätzlich α-Interferon und/oder Ribavirin verwendet wird.

7. Verwendung nach einem der Ansprüche 1 bis 6, **dadurch gekennzeichnet,** daß es sich bei der Erkrankung um chronische Hepatitis C und/oder ein Leberzellkarzinom handelt.
